# FASCICULE DE BREVET EUROPEEN

(11) **EP 1 895 993 B1**
(45) Date de publication et mention de la délivrance du brevet: **26.02.2014**
(21) Numéro de dépôt: 05762556.8
(22) Date de dépôt: 31.05.2005
(51) Int. Cl.: A61K 9/70, A61K 49/00, A61B 10/00

(54) **PATCH DOTE DE PROPRIETE ELECTROSTATIQUE POUR DEPISTAGE D'ALLERGENES, ET DE SON APPLICATEUR**
ELEKTROSTATISCHES PFLASTER FÜR DIE ALLERGEN-UNTERSUCHUNG UND APPLIKATOR DAFÜR
ELECTROSTATIC PATCH FOR ALLERGEN SCREENING AND APPLICATOR FOR SAME

(43) Date de publication de la demande: 12.03.2008
(73) Titulaire: DBV Technologies, 92100 Boulogne Billancourt (FR)
(72) Inventeur: DUPONT, Bertrand, F-13100 Aix En Provence (FR); BENHAMOU, Pierre-Henri, F-75015 Paris (FR)
(74) Mandataire: Becker, Philippe
(86) Numéro de dépôt international: PCT/FR2005/050397
(87) Numéro de publication internationale: WO 2006/128981

(56) Documents cités:
- EP-A- 0 976 396
- WO-A-98/31315
- WO-A-02/071950

## Description

L'invention a pour objet un patch dermique comprenant un support doté de propriétés électrostatiques présentant une surface d'adsorption augmentée.

L'invention a également pour objet un nouveau type de dispositif cutané. Plus précisément, elle a pour objet un dispositif intégrant un patch et un applicateur, celui-ci se séparant du patch à l'instant où ledit patch, sur lequel est déposée la substance destinée à être délivrée sur la surface de la peau, est appliqué sur ladite peau.

Dans la suite de la description, le patch dermique et le nouveau type de dispositif cutané sont plus particulièrement décrits en relation avec le dépistage de l'état de sensibilisation d'un sujet à un allergène donné, alimentaire, respiratoire, contact, etc, bien que toute autre application puisse être envisagée.

Deux types de tests sont utilisés pour détecter l'état dé sensibilisation vis-à-vis d'allergènes sont aujourd'hui commercialisés.

Il s'agit tout d'abord des tests connus sous la dénomination "Finn Chambers^{®}". Ce test est constitué d'un patch adhésif sur lequel sont collées de petites coupelles métalliques en aluminium au fond desquelles est simplement déposé, en l'absence de toute fixation, un disque de cellulose. En pratique, la solution d'allergène est soit préparée extemporanément, soit disponible dans le commerce et déposée sur le disque cellulosique au moment de l'utilisation. Les tests peuvent donc contenir plusieurs coupelles remplies chacune d'allergènes différents.

Ce type de test présente un certain nombre d'inconvénients, dont celui essentiel de devoir être préparé au moment de la pose avec tous les risques liés à la manipulation (contamination d'une coupelle par débordement, fausse manoeuvre, contacts avec le support adhésif, dosage aléatoire du principe actif etc..). De plus, l'absence de solidarisation du support métallique avec le disque cellulosique qui est simplement posé, peut être à l'origine de déplacement au moment de l'apposition dudit patch sur la peau. Il s'ensuit que l'adhésif d'une part, et le disque cellulosique d'autre part, qui présentaient probablement à l'origine les conditions requises d'inertie vis-à-vis respectivement, de la peau et de l'allergène, perdent au moment de leur utilisation, ces propriétés essentielles d'inertie.

Une application particulière de ce type de test, décrite dans le document US-A-4 836 217 est connue sous la dénomination "True test^{®}". L'originalité du True test^{®} par rapport au Finn chambers^{®} est d'utiliser un gel incorporant directement l'allergène, le gel étant déposé directement dans la coupelle métallique du Finn Chambers^{®}. Ce type de test est réservé aux allergènes de contacts principalement de nature non organique, ne posant à priori pas de problèmes de conservation dans un gel au contraire des composés organiques qui risquent de se modifier au cours du temps ou au contact d'un excipient quel qu'il soit.

En d'autres termes, le premier problème que se propose de résoudre l'invention est de développer un dispositif cutané :
- qui soit exempt de tout risque de contamination, que ce soit au moment de sa fabrication ou au moment de sa mise en oeuvre par l'utilisateur final,
- dont la partie adhésive, lorsqu'elle existe ne soit pas touchée, en quelque endroit que ce soit, de manière à assurer une adhésion parfaite et de longue durée du patch
- qui permette de conserver le principe actif sans modification de sa fonction allergénique.

Le second problème que se propose de résoudre l'invention est de développer un dispositif cutané qui puisse recevoir, en quantité suffisante aussi bien des allergènes liquides ou en suspension que des allergènes sous forme solide, en poudre sèche.

Le document WO02/30281 décrit un système d'application de patch transdermique, c'est-à-dire un système invasif. Plus précisément, le système est constitué d'un bouton poussoir à l'extrémité duquel le patient positionne au moment de son application, un patch transdermique c'est-à-dire un patch associé à une plaque métallique munie de pointes permettant de perforer la peau. Ce système, outre le fait qu'il concerne les patchs transcutanés ou transdermiques et non les patchs dermiques, ne résout pas le problème du risque de contamination puisque l'utilisateur manipule le patch avant son application.

Le document WO 02/089717 décrit un patch dermique dont la face destinée à être au contact de la peau est protégée par un liner rigide, le liner étant solidaire de deux languettes retirées au moment de l'utilisation. De même que précédemment, le patch et notamment sa partie adhésive, risque d'être contaminé au moment de l'utilisation. En outre, on peut s'attendre à ce qu'une partie de la substance active reste à la surface du liner une fois retiré, modifiant ainsi le dosage d'origine.

Le document US-A-5236455 décrit un système permettant la mise en place de « patch » pour remédier aux perforations de tympan. En pratique, le patch est positionné au bout de l'extrémité distale d'une tige formant cloche, le patch étant maintenu en position par mise sous vide de la cavité formée par la cloche. Le système nécessite donc une installation complexe de pompe à vide. Le patch proprement dit se présente sous la forme d'une simple membrane ayant pour vocation de boucher mécaniquement le trou et ne contient donc aucune substance active.

Le Demandeur a réussi à résoudre l'ensemble des problèmes en associant de manière réversible, un patch à un applicateur, la substance destinée à être appliquée sur la peau, étant solidaire du patch au moment de l'utilisation.

En d'autres termes, l'invention concerne un dispositif cutané non invasif à usage unique, qui se caractérise en ce qu'il comprend un patch constitué d'un support présentant à sa surface au moins une substance destinée à être délivrée sur la surface de la peau, le dos du patch étant fixé de manière réversible sur un applicateur, lequel est désolidarisé du patch après application et adhésion dudit patch sur la peau.

En d'autres termes, grâce à cette structure originale, l'utilisateur final, au moment de l'utilisation, n'a qu'à saisir le dispositif cutané au moyen de son applicateur et à déposer le patch actif sur la peau. Il n'y a donc aucun contact entre le patch actif et les doigts de l'utilisateur. Par ailleurs, le système proposé est un système non invasif en ce sens qu'il n'y a pas perforation de la peau par l'applicateur et/ou le patch.

Selon une première caractéristique, le dispositif cutané combine un patch présentant une cavité dans laquelle est maintenue la substance et un applicateur présentant un logement apte à recevoir ladite cavité. Au niveau de la cavité, la substance et le patch ne sont donc pas en pression sur la peau, ce qui permet à la sueur et à l'air de circuler et d'éviter l'effet de macération, capable de provoquer des rougeurs et des irritations. En outre, la circulation sanguine n'est pas altérée au niveau de la zone considérée.

Par ailleurs, pour que le patch puisse adhérer sur la peau, tout ou partie de la surface dudit patch destinée à être appliquée sur la peau est enduite de matière adhésive.

Selon une caractéristique essentielle de l'invention, la fixation du patch à la surface de l'applicateur est réversible. Tout moyen de fixation réversible peut être envisagé.

Dans un premier mode de réalisation, la fixation réversible du patch sur l'applicateur est obtenue au moyen d'une colle dite « repositionnable » appliquée sur tout ou partie du dos du patch et/ou de la surface de l'applicateur sur laquelle est fixé le patch.

Dans un second mode de réalisation, l'applicateur et le patch présentent des cavités de forme complémentaire, l'une mâle (le patch) et l'autre femelle (l'applicateur), la fixation réversible du patch sur l'applicateur étant obtenue par ajustement des deux cavités entre elles. L'ajustement peut par exemple résulter des dimensions de l'élément femelle et de celles de l'élément mâle.

La fixation réversible peut aussi être obtenue au moyen de petites dents ou protubérances pratiquées sur le pourtour de la partie femelle, lesdites dents ou protubérances venant enserrer la partie mâle constituée par le patch qui est élastique. Dans les deux cas, l'utilisation d'un matériau élastique pour le patch et/ou pour l'applicateur favorise l'ajustement et donc la fixation réversible de l'un sur l'autre.

Selon un autre mode de réalisation, l'applicateur, partie femelle de l'assemblage, est réalisé en matière plastique (polypropylène par exemple) et comporte des pattes flexibles qui se déforment légèrement lors du montage du patch et le maintiennent en exerçant sur sa périphérie une légère pression.

Dans un autre mode de réalisation, le patch, fabriqué d'une seule pièce dans un matériau élastique, a une forme qui rend l'ajustement facile :
- soit par déformation,
- soit par la présence d'une gorge où vient se loger la partie complémentaire de l'applicateur.

Pour éviter tout risque de contact entre les doigts de l'utilisateur et le patch, l'applicateur est muni d'un moyen de préhension.

Le patch en tant que tel peut être de différente nature en fonction de la substance qu'il incorpore.

Selon un premier mode de réalisation particulièrement avantageux, la substance se présente sous forme sèche, en particulier notamment sous forme de particules individualisées ou agglomérées.

Dans ce cas, le patch est constitué d'un support doté de propriétés électrostatiques, de sorte que la substance, sous forme de particules individualisées ou agglomérées, est maintenue au contact de la surface du support par des forces électrostatiques.

Ce type de patch a été développé par le Demandeur et est précisément décrit dans le document WO 02/071950 en relation avec une «substance biologiquement active", c'est à dire une substance à visée diagnostique, thérapeutique, cosmétique ou préventive ; en l'espèce un allergène. L'un des avantages de ce patch est qu'il permet de fixer directement, en l'absence de tout adjuvant, les allergènes sous forme pure, native, entière ou fractionnée.

Contrairement au patch de WO 02/071950 A , les micro-reliefs présents sur le patch de l'invention ne sont pas générés par les particules de la substance déposée mais lors du processus de fabrication du support du patch sur lequel est ensuite effectué le dépôt.

Lorsque l'allergène se présente sous forme liquide, tel que par exemple le lait, celui-ci est séché ou lyophilisé, puis broyé pour obtenir une forme poudre.

Dans un autre mode de réalisation, la forme liquide n'est pas séchée mais adsorbée sur une substance inerte. Plus précisément, la substance active est, dans ce cas, déposée sous forme de goutte sur le support électrostatique sur lequel est adsorbée la substance sèche. Dans ce cas, la substance sèche est une substance inerte apte, d'une part à étaler la goutte de liquide contenant la substance biologiquement active sur le support et, d'autre part, apte à se gélifier lors du contact avec ce liquide. En pratique, la substance inerte est choisie dans le groupe comprenant les dérivés du dextran ou les dérivés de celluloses.

Dès lors, l'invention a également pour objet un patch dermique constitué d'un support électrostatique destiné à être appliqué sur la peau et sur lequel est maintenue, par le biais de forces électrostatiques, au moins une substance se présentant sous forme de particules individualisées ou agglomérées, caractérisé en ce que la substance est une substance inerte, améliorant la mouillabilité du support d'une part et étant apte, d'autre part à se gélifier au contact d'une substance biologiquement active liquide, laquelle se présente sous forme pure ou en suspension.

Comme déjà dit, le support est avantageusement un support électrostatique.

Par l'expression "support électrostatique", on désigne tout support fabriqué en un matériau susceptible d'accumuler des charges électrostatiques à sa surface et de les conserver en développant ainsi des forces de maintien, notamment par frottement, chauffage et conditionnement électrique, ionisation ou toute autre technique connue de l'homme du métier.

Le support ou patch électrostatique constitue alors ce qu'on appelle un «électret» ; il présente, à sa surface, un champ électrique permanent, de signe positif ou négatif, qui peut être mis en évidence par des moyens de mesure appropriés et connus de l'homme de métier.

Les charges apparaissant d'un côté ou de l'autre de la surface du support peuvent être positives ou négatives, en fonction du matériau constituant ledit support et du moyen utilisé pour les faire apparaître. Dans tous les cas, les charges positives ou négatives réparties sur la surface du support sont à l'origine de forces d'attraction sur des matériaux conducteurs ou non, dans le cas d'espèce de l'allergène sous forme de particules individualisées ou agglomérées. Il peut également arriver que les particules soient ionisées, ce qui peut provoquer alors le même type de force d'attraction électrostatique entre les particules et le support. L'attraction électrostatique peut aussi résulter d'une polarisation des particules par influence, du fait de la présence du champ électrique.

En pratique, tout matériau peut être utilisé comme support pourvu qu'il ait les qualités électrostatiques requises. En particulier, le support est constitué de verre ou d'un polymère choisi dans le groupe comprenant les plastiques cellulosiques (CACP), le polychlorure de vinyle, les polypropylènes, les polystyrènes, les polycarbonates, les polyacryliques, les polyoléfines, les polyesters, les polyéthylènes, les éthylènes/acrylate de vinyle (EVA).

Dans le mode de réalisation selon lequel le support électrostatique présente une cavité, la cavité peut être alors formée directement dans le support par formage à chaud ou à froid (thermoformage ou injection plastique).

Dans un autre mode de réalisation, la cavité est obtenue par l'association d'un premier support électrostatique correspondant au fond de la cavité, et d'un second support destiné à être appliqué sur la peau, les supports étant solidarisés au niveau de leur périphérie, par le biais d'un matériau de faible épaisseur, en pratique comprise entre 1 et 2 mm, par exemple en forme d'anneau si la cavité est circulaire. Dans ce cas, la fixation réversible du patch sur son l'applicateur est obtenue par ajustement.

Dans un mode de réalisation avantageux, la surface du support électrostatique n'est pas lisse mais présente des reliefs. La présence de micro-reliefs à la surface du support engendre, par rapport à un support lisse, la formation de petits creux dans lesquels vient se loger l'allergène, permettant ainsi d'augmenter la surface disponible et donc la quantité d'allergène présente sur le support. L'allergène maintenu dans les creux est ensuite progressivement relargué au niveau de la surface de la peau. Bien entendu, les reliefs sont de taille très faible, de l'ordre, en terme de profondeur, de quelques micromètres à quelques dixièmes de millimètres.

Dès lors, l'invention concerne également un patch, en particulier un patch dermique se présentant sous la forme d'un support doté de propriétés électrostatiques, recouvert ou étant susceptible d'être recouvert d'au moins une substance, avantageusement une substance biologiquement active, notamment un allergène destinée à être appliquée sur la peau, ledit patch se caractérisant en ce que la surface du support, en contact avec la substance, présente des micro reliefs.

Les micro reliefs peuvent avoir des formes variables et être obtenus de diverses manières.

Dans un premier mode de réalisation, les reliefs sont obtenus à partir du support proprement dit, par exemple par formage à chaud. Les reliefs créent des creux et des bosses pouvant avoir des formes variables, carrées, hexagonales, circulaires, radiales, etc..

Dans un second mode de réalisation, les micro-reliefs sont obtenus par superposition, sur le support, d'une ou plusieurs feuilles soudées ou collées au moyen d'un film adhésif sur ses deux faces, le support alors appelé « backing » étant étanche et lisse, les feuilles étant percées de multiples trous qui permettant à l'allergène de venir se loger entre les feuilles et, également, dans les trous eux-mêmes.

Dans un troisième mode de réalisation, les micro-reliefs sont obtenus par fixation sur le support, de multiples filaments ou « poils », très fins et très courts et en très grande quantité qui forment une sorte de tapis apte à recevoir et à maintenir par capillarité la substance, notamment l'allergène déposé. Dans le cas où le support est un support électrostatique, la présence d'arêtes ou les faibles dimensions des poils peut augmenter l'attraction électrostatique par effet de pointe.

La présence de micro-reliefs permet de préparer des patchs dont le support incorpore plusieurs substances biologiquement actives. Ces substances peuvent être introduites dans le support à des profondeurs variables, de sorte que le premier principe actif est d'abord libéré grâce la sudation avant que le deuxième principe actif, situé dans l'épaisseur du support ne soit libéré. L'un des principes actifs joue par exemple le rôle d'adjuvant agissant sur les propriétés du deuxième (dilution, révélation de son action biologique etc.). Il peut également agir sur la peau en favorisant le passage trans-cutané, en augmentant la vascularisation locale ou en modifiant plus généralement les qualités physiques ou chimiques locales.

Dans le mode de réalisation multi-couche du « backing », la disposition du principe actif en plusieurs couches permet de régler la libération dudit principe actif. Ce type de patch permet ainsi de contrôler à la fois la quantité libérée et le débit de libération du principe actif.

Lorsque la substance biologiquement active, notamment l'allergène se présente sous forme liquide ou en suspension et que l'on souhaite l'utiliser non pas sous forme de poudre, mais dans sa forme naturelle, alors l'allergène est incorporé dans le patch extemporanément. Dans ce cas, le patch doit être capable d'adsorber l'allergène sous forme liquide.

Dans le mode de réalisation décrit précédemment, une substance inerte sèche, sous forme poudre, est maintenue sur le support par le biais de forces électrostatiques, la substance étant capable de se gélifier au contact de la substance active sous forme liquide. Dans ce cas, la substance active est mise au contact de la substance inerte immédiatement avant application du patch. La substance inerte se gélifie et libère progressivement la substance active qu'elle contient.

Selon un second mode de réalisation, le support n'est pas un support électrostatique et se présente sous la forme d'un tissé ou d'un non tissé, mono ou multicouches à base de fibres naturelles, telles que la cellulose ou synthétiques, tels que le polypropylène, seules ou en mélange, le grammage du support étant compris entre 25-120 g/m².

Dans un troisième mode de réalisation, le support se présente sous la forme d'un film à base de polymères ayant subi un traitement physico-chimique qui augmente la mouillabilité de la surface adsorbante permettant ainsi à la goutte déposée de s'étaler sur le patch.

Dans un quatrième mode de réalisation le support est un support, avantageusement en matière plastique, notamment sous forme d'un film, ayant éventuellement subi un traitement physico-chimique améliorant sa mouillabilité et présentant des reliefs dont la forme et le procédé d'obtention sont identiques à ce qui a été décrit concernant les allergènes sous forme de poudre.

Contrairement aux allergènes secs qui peuvent être incorporés directement au moment de la fabrication du dispositif cutané, l'allergène liquide est adsorbé sur le patch au moment de l'utilisation. Pour ce faire, le dispositif cutané est muni d'un couvercle coopérant avec la surface de l'applicateur permettant de protéger le patch pendant le stockage. Dans ce cas, le couvercle présente un orifice permettant de passage de l'allergène, le couvercle étant retiré seulement une fois l'allergène adsorbé sur le patch.

Un autre avantage du couvercle est de protéger la surface adhésive du patch (lorsqu'elle existe) jusqu'au moment de la pose dudit patch, dans la mesure où cette dernière n'est pas revêtue d'un film de protection pelable.

Bien entendu, le patch et l'applicateur peuvent avoir des formes variables. Avantageusement, patch et applicateur sont de forme circulaire.

Pour rendre le dispositif cutané exempt de toute possibilité de contamination pendant son stockage, celui-ci est maintenu dans un blister qui constitue une enceinte fermée hermétiquement, avantageusement au moyen d'un opercule pelable.

L'invention et les avantages qui en découlent ressortiront mieux des exemples de réalisation suivants, à l'appui des figures annexées.
La figure 1 représente une vue éclatée du dispositif cutané de l'invention.
La figure 2 représente l'embase vue en coupe
La figure 3 représente un patch susceptible d'incorporer une substance biologiquement active.
La figure 4 représente un exemple de conditionnement du dispositif cutané de l'invention.

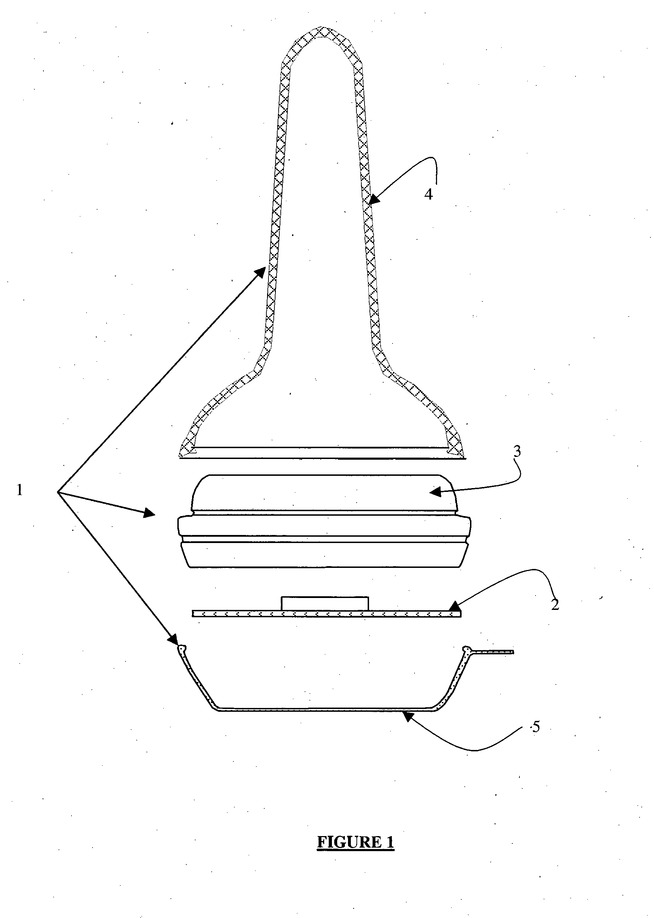

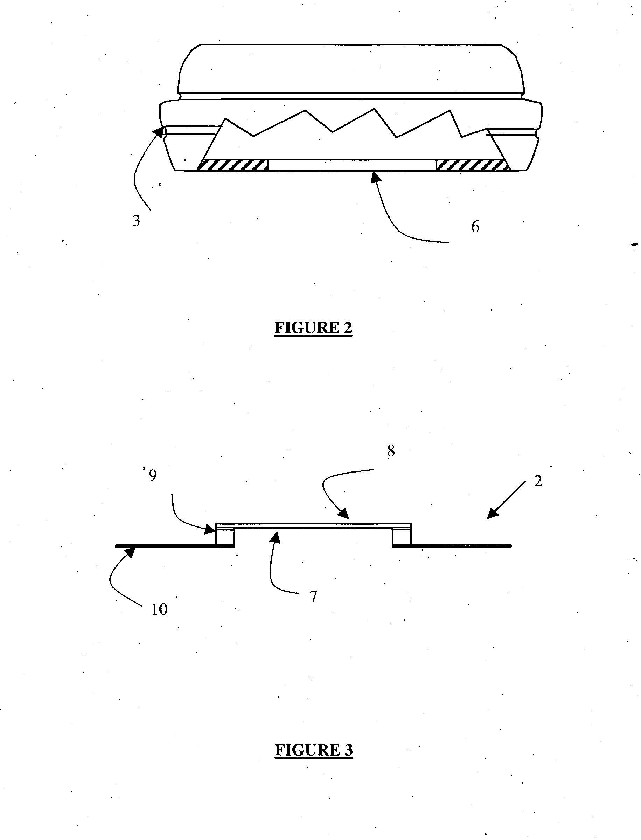

Conformément à l'invention, le dispositif cutané combine un applicateur (1) avec un patch destiné à contenir une substance biologiquement active (2). Dans le mode de réalisation tel que représenté sur la figure 1, l'applicateur est muni de trois pièces, respectivement une embase (3), un moyen de préhension (4) et un couvercle (5), les trois pièces présentant une section circulaire. Le moyen de préhension présente une taille d'environ 50 mm de manière à pouvoir être saisie facilement par les doigts de l'utilisateur. En pratique, cette pièce creuse est réalisée en matière plastique. Elle s'enclipse sur l'embase (3) de forme correspondante. Plus précisément, cette embase (3) représentée en coupe sur la figure 2, présente un logement (6) dans laquelle vient se loger la cavité correspondante (7) du patch (2). Enfin, une fois le patch positionné dans son logement, un couvercle (5) vient s'engager également par clipsage sur l'embase (3) de manière à protéger le patch et éventuellement la matière active qu'il contient pendant le stockage et au moment de l'utilisation. En pratique, le diamètre de l'embase est de 31 mm.

Le patch (2) est plus précisément représenté en coupe sur la figure 3. Dans ce mode de réalisation particulier, le fond (8) de la cavité (7) au moins est réalisé en un matériau électrostatique, en l'espèce de l'acétate de cellulose. Le fond de la cavité est séparé du patch (2) en tant que tel, au moyen d'une mousse (9) destinée à créer la cavité (7). Le patch (2) est quant à lui réalisé en un matériau plastique recouvert d'un adhésif (10) sur tout ou partie de la surface destinée à être appliquée sur la peau. Pour permettre la fixation réversible du patch (2) sur la surface de l'embase (3), le diamètre de la cavité (7) de même que celui de l'anneau de mousse (9) est choisi de manière à venir s'ajuster dans la cavité (6).

Lorsque la cavité contient en tant que substance biologiquement active un allergène sec, alors cet allergène est incorporé au moment de la fabrication du dispositif cutané. En revanche, lorsqu'il s'agit d'un allergène liquide, celui-ci est incorporé extemporanément par un orifice non représenté prévu à la surface du couvercle (5).

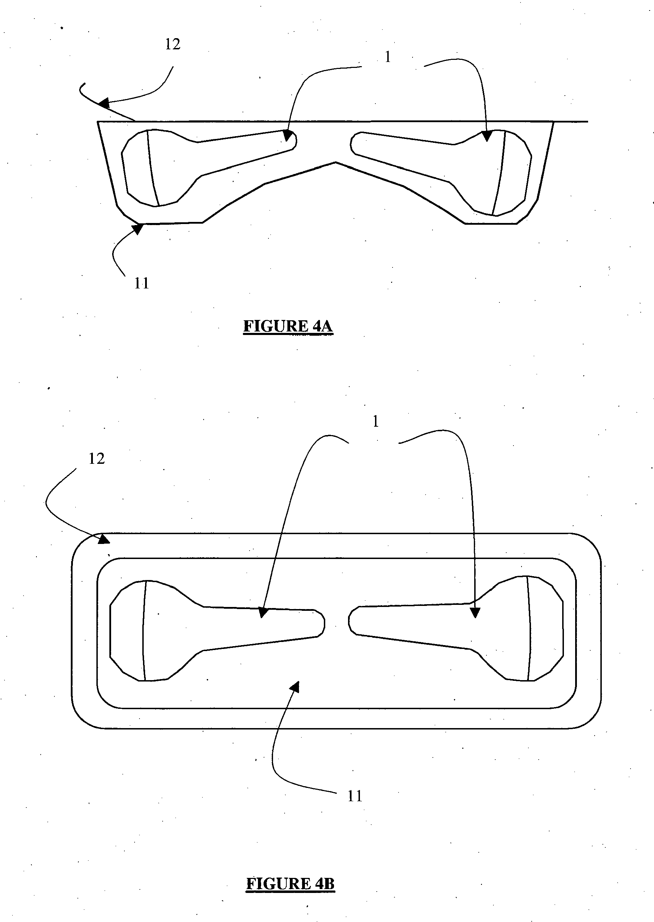

L'invention concerne également les dispositifs de stockage de dispositif cutané représentés sur la figure 4 en vue de dessus (figure 4a) et en coupe (figure 4b).

Plus précisément, le dispositif de stockage se présente sous forme d'un blister (11) renfermant, dans le mode de réalisation tel que représenté, deux dispositifs cutanés et recouvert d'un opercule pelable (12). Lors de la mise en oeuvre, l'utilisateur disposera donc d'au moins une boîte, voire plusieurs boîtes dans l'hypothèse où plusieurs allergènes distincts sont testés. Une fois le blister (11) ouvert, l'utilisateur retire le dispositif cutané en le saisissant par le moyen de préhension (4). Le couvercle (5) est ensuite retiré par déclipsage, puis le dispositif cutané directement appliqué à la surface de la peau, par exemple la peau du bébé. La même manoeuvre est répétée pour le second dispositif cutané et ainsi de suite.

Lorsque l'allergène se présente sous forme liquide, le couvercle (5) du dispositif cutané présente un orifice permettant le passage de l'allergène préparé extemporanément. Le couvercle est ensuite retiré puis le dispositif cutané appliqué sur la peau de l'utilisateur.

Les avantages de l'invention ressortent bien de la description qui précède. On notera en particulier l'absence totale de contamination du patch par l'utilisateur ainsi que sa capacité à recevoir tout type de substance biologique, en particulier d'allergène sous forme sèche ou liquide.

## Revendications

1. Patch dermique se présentant sous la forme d'un support doté de propriétés électrostatiques, recouvert ou étant susceptible d'être recouvert d'au moins une substance destinée à être appliquée sur la peau, ledit patch **se,caractérisant en ce que** la surface du support, en contact avec la substance, présente des micro-reliefs, sur lesquels ladite substance est déposée.

2. Patch selon la revendication 1, **caractérisé en ce que** les micro-reliefs sont obtenus à partir du support proprement dit, par formage à chaud.

3. Patch selon la revendication 1, **caractérisé en ce que** les micro-reliefs sont obtenus par superposition, sur le support, d'une ou plusieurs feuilles soudées ou collées au moyen d'un film adhésif sur ses deux faces, le support étant étanche et lisse, les feuilles étant percées de multiples trous.

4. Patch selon la revendication 1, **caractérisé en ce que** les micro-reliefs sont obtenus par fixation sur le support, de multiples filaments, formant un tapis apte à recevoir et à maintenir par capillarité la substance déposée.

5. Patch selon l'une des revendications précédentes, **caractérisé en ce que** le support est constitué de verre ou d'un polymère choisi dans le groupe comprenant les plastiques cellulosiques (CA, CP), le polychlorure de vinyle (PVC), les polypropylènes, les polystyrènes, les polyuréthanes, les polycarbonates, les polyacryliques les polyoléfines, les polyesters, les polyéthylènes, les éthylènes/acrylate de vinyle (EVA).

6. Patch selon la revendication 1, **caractérisé en ce que** la substance se présente sous forme de particules individualisées ou agglomérées.

7. Patch selon la revendication 6, **caractérisé en ce que** la substance est une substance biologiquement active à visée diagnostique, thérapeutique, cosmétique ou préventive, notamment un allergène.

8. Patch selon la revendication 6, **caractérisé en ce que** la substance est une substance inerte apte à se gélifier au contact d'une substance biologiquement active à visée diagnostique, thérapeutique, cosmétique ou préventive, se présentant sous forme liquide.

9. Dispositif cutané non invasif à usage unique, **caractérisé en ce qu'**il comprend un patch selon l'une quelconque des revendications 1 à 8, le dos du patch étant fixé de manière réversible sur un applicateur (1), lequel est désolidarisé du patch après application et adhésion dudit patch (2) sur la peau.

10. Dispositif cutané selon la revendication 9, **caractérisé en ce que** :
- le patch (2) présente une cavité (7) dans laquelle est maintenue la substance
- l'applicateur (1) présente un logement (6) apte à recevoir ladite cavité.

11. Dispositif cutané selon l'une des revendications 9 ou 10, **caractérisé en ce que** tout ou partie de la surface du patch (2) destinée à être appliquée sur la peau est enduite de matière adhésive.

12. Dispositif cutané selon la revendication 10, **caractérisé en ce que** la fixation réversible du patch (2) avec l'applicateur (1) est obtenue par ajustement de la cavité (7) et du logement (6).

13. Dispositif cutané selon la revendication 9, **caractérisé en ce que** l'applicateur est muni d'un moyen de préhension (4).

14. Dispositif cutané selon la revendication 10, **caractérisé en ce qu'**il comprend en outre un couvercle (5) coopérant avec la surface de l'applicateur (1), ledit couvercle (5) présentant un orifice permettant le passage de la substance lorsque celle-ci se présente sous la forme d'une substance biologiquement active liquide préparée extemporanément.

15. Dispositif de stockage de dispositif cutané, **caractérisé en ce qu'**il se présente sous la forme d'une enceinte (11) dans laquelle est stocké au moins un dispositif cutané objet de l'une des revendications 10 à 15, ladite enceinte étant fermée hermétiquement (12).

## Patentansprüche

1. Hautpflaster, das einen Träger mit elektrostatischen Eigenschaften umfasst, der mit wenigstens einer Substanz beschichtet ist oder beschichtet sein kann, die auf die Haut appliziert werden soll, wobei besagtes Pflaster **dadurch gekennzeichnet ist, dass** die Oberfläche des Trägers, die in Kontakt mit der Substanz steht, Mikroreliefs aufweist, auf denen besagte Substanz aufgetragen ist.

2. Pflaster gemäß Anspruch 1, **dadurch gekennzeichnet, dass** die Mikroreliefs eigentlich durch Wärmeverformung des Trägers erhalten werden.

3. Pflaster gemäß Anspruch 1, **dadurch gekennzeichnet, dass** die Mikroreliefs auf dem Träger durch Superposition einer oder mehreren Folien, die mithilfe eines Doppelklebefilms verschweißt oder verklebt sind, erhalten werden, wobei der Träger undurchlässig und glatt ist und die Folien mit mehreren Löchern perforiert sind.

4. Pflaster gemäß Anspruch 1, **dadurch gekennzeichnet, dass** die Mikroreliefs erhalten werden durch Fixierung mehrerer Fasern auf dem Träger, die eine geeignete Faserschicht bilden, die zur Aufnahme und Adsorption der aufgetragenen Substanz durch Kapillarwirkung geeignet ist.

5. Pflaster gemäß einem der vorhergehenden Ansprüche, **dadurch gekennzeichnet, dass** der Träger aus Glas oder einem Polymer besteht, ausgewählt aus der Gruppe, umfassend Cellulose-Kunststoffe (CA, CP), Polyvinylchlorid (PVC), Polypropylene, Polystyrole, Polyurethane, Polycarbonate, Polyacrylate, Polyolefine, Polyester, Polyethylene, Ethylen/Vinylacrylate (EVA).

6. Pflaster gemäß Anspruch 1, **dadurch gekennzeichnet, dass** die Substanz in Form von einzelnen oder agglomerierten Partikeln vorliegt.

7. Pflaster gemäß Anspruch 6, **dadurch gekennzeichnet, dass** die Substanz eine biologisch aktive Substanz für diagnostische, therapeutische, kosmetische oder präventive Zwecke ist, insbesondere ein Allergen ist.

8. Pflaster gemäß Anspruch 6, **dadurch gekennzeichnet, dass** die Substanz eine inerte Substanz ist, die für eine Gelbildung bei Kontakt mit einer biologisch aktiven Substanz für diagnostische, therapeutische, kosmetische oder präventive Zwecke geeignet ist, wobei sie in flüssiger Form vorliegt.

9. Nichtinvasive Hautvorrichtung zur Einmalverwendung, **dadurch gekennzeichnet, dass** sie ein Pflaster gemäß einem der Ansprüche 1 bis 8 umfasst, wobei die Rückseite des Pflasters ablösbar auf einem Applikator (1) befestigt ist, welcher nach Applikation und Aufkleben besagten Pflasters (2) auf der Haut entfernt wird.

10. Hautvorrichtung gemäß Anspruch 9, **dadurch gekennzeichnet, dass**:
- das Pflaster (2) einen Hohlraum (7) aufweist, in dem die Substanz adsorbiert ist
- der Applikator (1) einen Einschnitt (6) aufweist, der zur Aufnahme besagten Hohlraums geeignet ist.

11. Hautvorrichtung gemäß einem der Ansprüche 9 oder 10, **dadurch gekennzeichnet, dass** die gesamte oder ein Teil der Oberfläche des Pflasters (2), das auf die Haut appliziert werden soll, mit Klebstoff beschichtet ist.

12. Hautvorrichtung gemäß der Anspruch 10, **dadurch gekennzeichnet, dass** die ablösbare Befestigung des Pflasters (2) mit dem Applikator (1) durch Justieren des Hohlraums (7) und des Einschnitts (6) erhalten wird.

13. Hautvorrichtung gemäß Anspruch 9, **dadurch gekennzeichnet, dass** der Applikator mit einem Greifmittel (4) versehen ist.

14. Hautvorrichtung gemäß Anspruch 10, **dadurch gekennzeichnet, dass** sie außerdem eine Abdeckung (5) zusammen mit der Oberfläche des Applikators (1) umfasst, wobei besagte Abdeckung (5) einen Durchlass aufweist, der den Durchtritt der Substanz erlaubt, wenn diese in Form einer extemporan zubereiteten flüssigen biologisch aktiven Substanz vorliegt.

15. Vorrichtung zum Aufbewahren der Hautvorrichtung, **dadurch gekennzeichnet, dass** sie in Form einer Schutzhülle (11) vorliegt, in dem wenigstens eine Hautvorrichtung, die Gegenstand eines der Ansprüche 10 bis 15 ist, aufbewahrt wird, wobei besagte Schutzhülle hermetisch verschlossen ist (12).

## Claims

1. A skin patch which is in the form of a support with electrostatic properties, covered or coverable with at least one substance intended to be applied on the skin, wherein the surface of the support of said patch, in contact with the substance, has micro-reliefs, on which said substance is deposited.

2. Patch according to claim 1, wherein the micro-reliefs are obtained from the support itself, by hot moulding.

3. Patch according to claim 1, wherein the micro-reliefs are obtained by superposition, on the support, of one or several soldered or glued layers using a double-sided adhesive film, the support being waterproof and smooth, the layers being punched with multiple holes.

4. Patch according to claim 1, wherein the micro-reliefs are obtained by fixing multiple strands on the support, forming a carpet capable of receiving and maintaining the substance deposed via capillary action.

5. Patch according to any one of the preceding claims, wherein the support is constituted of glass or of a polymer selected from the group comprising cellulose plastics (CA, CP), polyvinyl chloride (PVC), polypropylenes, polystyrenes, polyurethanes, polycarbonates, polyacrylics, polyolefins, polyesters, polyethylenes, ethylene vinyl acrylate (EVA).

6. Patch according to claim 1, wherein the substance is in the form of individualised or agglomerated particles.

7. Patch according to claim 6, wherein the substance is a biologically active substance for diagnostic, therapeutic, cosmetic or preventive purposes, especially an allergen.

8. Patch according to claim 6, wherein the substance is an inert substance able to gel upon contact with a biologically active substance for diagnostic, therapeutic, cosmetic or preventive purposes, being in a liquid form.

9. Single-use non-invasive skin device comprising a patch according to any one of claims 1 to 8, the back of the patch being fixed in a reversible manner on an applicator (1), which is removed from the patch after application and adhesion of said patch (2) onto the skin.

10. Skin device according to claim 9, wherein:
- the patch (2) has a cavity (7) in which is maintained the substance;
- the applicator (1) has a housing (6) able to receive said cavity.

11. Skin device according to claim 9 or 10, wherein all or part of the surface of the patch (2) destined to be applied onto the skin is coated with an adhesive material.

12. Skin device according to claim 10, wherein the reversible fixation of the patch (2) with the applicator (1) is obtained by adjusting the cavity (7) and the housing (6).

13. Skin device according to claim 9, wherein the applicator is equipped with a means of prehension (4).

14. Skin device according to claim 10, further comprising a lid (5) cooperating with the surface of the applicator (1), said lid (5) having an orifice allowing the passage of the substance when the substance is in the form of the biologically active substance prepared extemporaneously.

15. Storage device for a skin device, wherein said storage device is in the form of an enclosure (11) in which is stored at least one skin device of any one of claims 10 to 15, said enclosure being hermetically closed (12).
